## Europäisches Patentamt
## European Patent Office
## Office européen des brevets

(19)

(11) Veröffentlichungsnummer: **0 013 748**
**B1**

(12) **EUROPÄISCHE PATENTSCHRIFT**

(45) Veröffentlichungstag der Patentschrift: **11.04.84**

(51) Int. Cl.³: **C 12 M 1/26, C 12 M 1/22**

(21) Anmeldenummer: **79105315.0**

(22) Anmeldetag: **21.12.79**

(54) Abklatschkulturaufnahmeeinheit.

(30) Priorität: **30.01.79 DE 2903400**

(43) Veröffentlichungstag der Anmeldung:
**06.08.80 Patentblatt 80/16**

(45) Bekanntmachung des Hinweises auf die Patenterteilung:
**11.04.84 Patentblatt 84/15**

(84) Benannte Vertragsstaaten:
**AT BE CH DE FR GB IT NL**

(56) Entgegenhaltungen:
**US - A - 2 030 344**
**US - A - 2 077 868**
**US - A - 3 141 400**
**US - A - 3 203 870**
**US - A - 3 751 341**

(73) Patentinhaber: **F. HOFFMANN-LA ROCHE & CO.**
**Aktiengesellschaft**
**CH-4002 Basel (CH)**

(72) Erfinder: **Jünger, Josef**
**Edmund Schweizerstrasse 1**
**D-7888 Rheinfelden (DE)**
Erfinder: **Wehrle, Herbert**
**Markgrafenstrasse 31**
**D-7889 Grenzach-Wyhlen (DE)**

(74) Vertreter: **Lederer, Franz, Dr. et al,**
**Patentanwälte Dr. Lederer Franz Meyer-Roxlau**
**Reiner F. Lucile-Grahn-Strasse 22**
**D-8000 München 80 (DE)**

Courier Press, Leamington Spa, England.

# 0 013 748

## Abklatschkulturaufnahmeeinheit

Die Erfindung betrifft eine Abklatschkulturaufnahmeeinheit, die dazu dient, die Keimbesiedlung von Oberflächen hinsichtlich der Erregerart, der Zahl und Schliesslich auch der Verteilung der Keime im Bereich einer Fläche zu erfassen, gegen die die Abklatschkulturaufnahmeeinheit angedrückt wird, so dass Keime abgenommen, d.h. "abgeklatscht" werden und dann in der Abktlatschkulturaufnahmeeinheit weiterwachsen und somit identifiziert und zugeordnet werden können.

Natchteilig bei den bekannten Anordnungen dieser Art (vgl. Fig. 1 und 2) ist es, dass der Agar-Nährboden, mit dem die Schale gefüllt ist, nach seinem Erkalten eine etwas konkave Oberfläche aufweist, so dass das Andrücken an ebene Flächen oder gerade Flächen, die in ihrer Ausdehnung grösser als der Durchmesser des Steges sind, nicht möglich ist, während im Gegenteil hierzu gerade eine nach Art eines Stempelkissens konvexe Oberfläche (vgl. Fig. 3) an und für sich erforderlich wäre, um das Andrücken an ebene oder gerade Flächen zu ermöglichen.

Aufgabe der Erfindung ist es daher, eine Abklatschkulturaufnahmeeinheit der eingangs genannten Art zu schaffen, die auch das Andrücken der Oberfläche des Agar-Nährbodens an Gegenstände erlaubt, die gerade bzw. eben sind.

Erfindungsgemäss wird diese Aufgabe gelöst durch eine Abklatschkulturaufnahmeeinheit mit einer Schale, die einen entlang ihres Randes umlaufenden Steg aufweist, wobei der innerhalb des Steges der Schale gebildete Raum zur Füllung mit Agar-Nährboden bestimmt ist bzw. damit gefüllt ist, dadurch gekennzeichnet, dass über den oberen Rand (12) nach oben hervorstehend eine weitere Umrandung (11) vorgesehen ist, dass der gesamte, durch den Steg (9) und die weitere Umrandung (11) gebildete Raum zur Füllung mit Agar-Nährboden (3) bestimmt ist bzw. damit angefüllt ist, und dass die weitere Umrandung (11) abnehmbar ist.

Die Erfindung betrifft sowohl gefüllte wie ungefüllte Einheiten.

Ein Ausführungsbeispiel der Erfindung wird im folgenden unter Bezugnahme auf die beigefügten Zeichnung beschrieben. Es stellen dar:

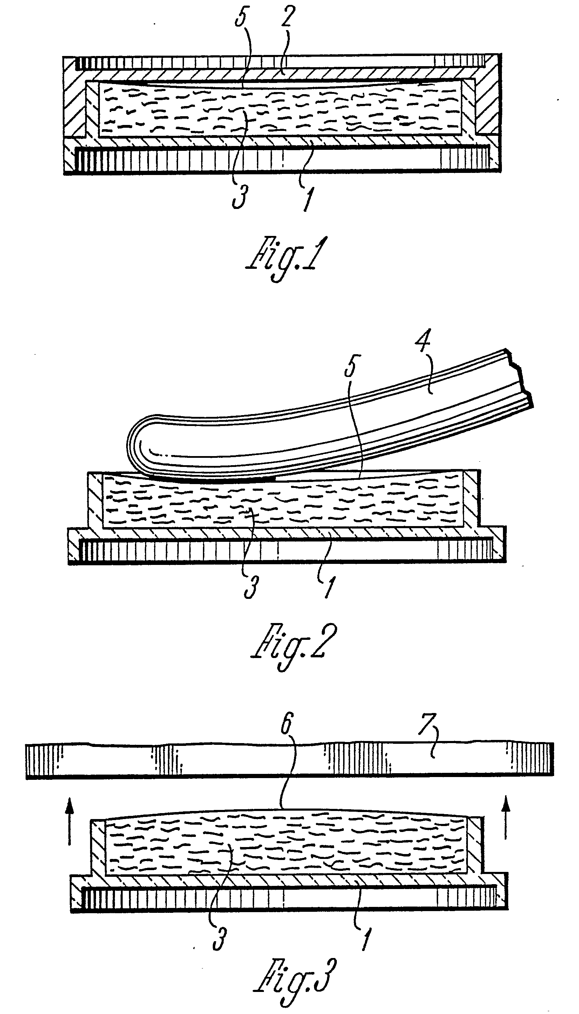

Figur 1 eine Abklatschkulturaufnahmeeinheit nach dem Stand der Technik;

Figur 2 die Verwendung einer Abklatschkulturaufnahmeeinheit gemass Fig. 1;

Figur 3 Darstellung einer wünschenswerten Ausbildung der Oberfläche des Agar-Nährbodens bei einer Abklatschkulturaufnahmeeinheit nach Fig. 1 oder 2;

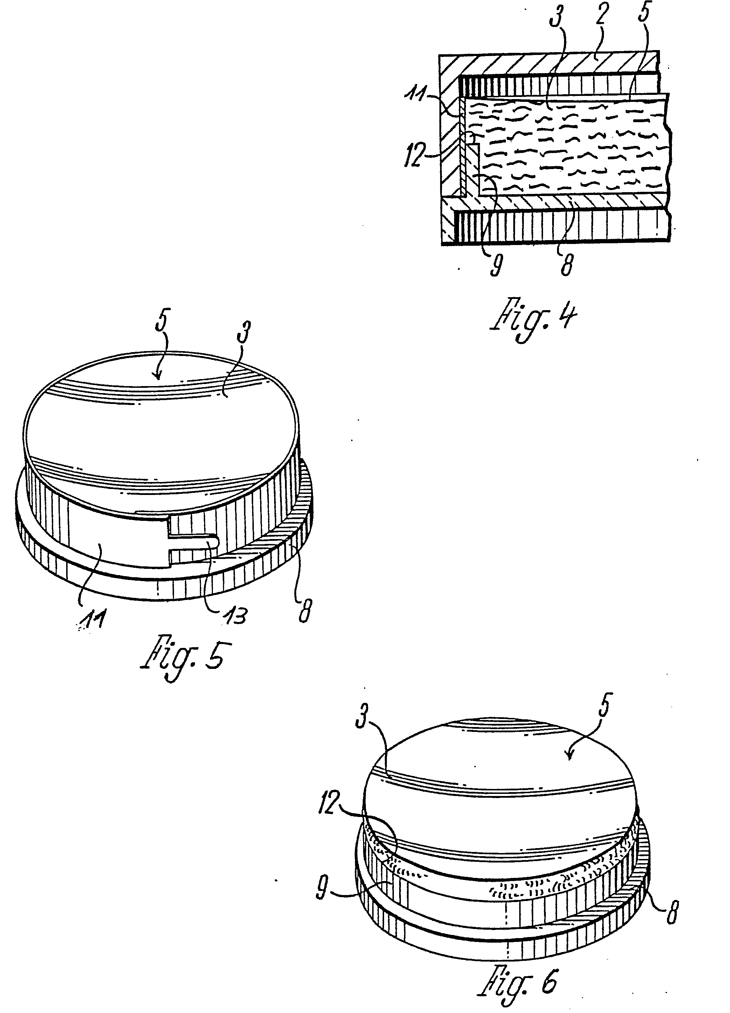

Figur 4 ein Ausführungsbeispiel der Erfindung;

Figur 5 das erwähnte Ausführungsbeispiel in perspektivischer Ansicht ohne Deckel;

Figur 6 das erwähnte Ausführungsbeispiel mit abgenommenem Abrisstreifen.

Die Abklatschkulturaufnahmeeinheit nach dem Stand der Technik, die in Fig. 1 und 2 dargestellt ist, besteht aus einer Schale 1 und einem Deckel 2. Die Schale 1 ist mit Agar-Nährboden 3 gefüllt. Die Verwendung erfolgt gemäss Fig. 2 derart, dass irgend eine Gegenstand, dessen Oberflächenbelegung mit Keimen erfasst werden soll, in Fig. 2 mit 4 bezeichnet, z.B. eine Türklinke, eine Beckenarmatur, ein Instrument, o. dgl., in die Oberfläche des Agar-Nährbodens eingedrückt wird. Dabei werden die auf dem Gegenstand 4 vorhandenen Keime, d.h. seine "bakterielle Verunreinigung" im Agar-Nährboden abgedrückt bzw. von ihm abgenommen, also "abgeklatscht" und somit — nach entsprechendem Wachstum auf dem Agar-Nährboden 3 — hinsichtlich der Erregerart (qualitativ), der Zahl (quantitativ) und schliesslich auch hinsichtlich der Verteilung auf der Oberfläche des Gegenstandes 4 (topographisch) erfassbar.

Das Problem bei derartigen Abklatschkulturaufnahmeeinheiten besteht nun darin, dass die Oberfläche in der in die Schale 1 eingebrachten Agar-Nährböden leicht konkar ist, wie dies in Fig. 1 und Fig. 2 angedeutet ist. Diese negative Meniskusform ergibt sich beim Erkalten des in die Schale 1 eingegossenen Agar-Nährbodens.

Um auch von ebenen Oberflächen oder von geraden Gegenständen, die länger als der Durchmesser der Schale 1 sind, einen guten Abdruck herzustellen, wäre nun erwünscht, dass gerade im Gegensatz zu diesen tatsächlichen Gegebenheiten die Oberfläche 6 eines Agar-Nährbodens 3, wie in Fig. 3 angedeutet, nicht konkav, sondern konvex wäre. Dann könnte ein Abdruck auch eines ebenen bzw. geraden Gegenstandes 7 genommen werden. Eine solche Oberfläche kann man jedoch beim Eingiessen des Agar-Nährbodens in eine Schale bzw. nicht einfach in einem einmaligen Vorgang erzielen. Wie bereits in der Einleitung dargelegt, ist es daher Aufgabe der Erfindung, eine Abklatschkulturaufnahmeeinheit zu finden, bei der ein Abdruck oder Abklatsch eines ebenen und/oder geraden Gegenstandes mit Abmessungen, die grösser als der Durchmesser der Schale sind, möglich wird.

Ein Ausführungsbeispiel gemäß vorliegendes Erfindung zeigen die Fig 4 bis 5. Hier ist der Steg 9 der Schale mit einem Abreissstreifen 11 umgeben, der höher als der Steg 9 ist. Ist der Agar-Nährboden 3 eingegeben, so kann vor der Benutzung der Abreissstreifen 11 mit Hilfe der an ihm vorgesehenen Lasche 13 (vgl. Fig. 10) abgerissen werden, so dass der Agar-Nährboden 3 oberhalb des oberen Randes 12 des Steges 9 stehen bleibt und auch wieder, selbst bei einer gewissen Konkavität der Oberfläche 5 desselben, leicht gegen einen ebenen und geraden Gegenstand zur Abnahme eines Abklatsches angedrückt werden kann.

# O 013 748

**Patentansprüche**

1. Abklatschkulturaufnahmeeinheit mit einer Schale die einen entlang ihres Randes umlaufenden Steg aufweist, wobei der innerhalb des Steges der Schale gebildete Raum zur Füllung mit Agar-Nährboden bestimmt ist bzw. damit gefüllt ist, dadurch gekennzeichnet, dass über den oberen Rand (12) nach oben hervorstehend eine weitere Umrandung (11) vorgesehen ist, dass der gesamte, durch den Steg (9) und die weitere Umrandung (11) gebildete Raum zur Füllung mit Agar-Nährboden (3) bestimmt ist bzw. damit angefüllt ist, und dass bei weitere Umrandung (11) abnehmbar ist.

2. Abklatschkulturaufnahmeeinheit nach Anspruch 1, dadurch gekennzeichnet, dass die weitere Umrandung als entfernbarer Streifen (11) ausgebildet ist.

3. Abklatschkulturaufnahmeeinheit nach Anspruch 2 dadurch gekennzeichnet, dass der entfernbare Streifen (11) als Abreissstreifen mit einer Lasche (13) versehen, ausgebildet ist.

**Revendications**

1. Unité de prélèvement par contact pour cultures comprenant une coupelle portant une nervure périphérique sur toute sa bordure, l'espace formé à l'intérieur de la nervure de la coupelle étant prévu pour être garni d'un milieu nutritif à la gélose ou étant garni d'un tel milieu, caractérisée en ce que l'on a prévu au-dessus du bord supérieur (12) une autre bordure faisant saillie vers le haut, en sorte que tout l'espace formé par la nervure (9) et la nouvelle bordure (11) est prévu pour être garni d'un milieu nutritif à la gélose (3) ou est garni d'un tel milieu, et en ce que la nouvelle bordure est démontable.

2. Unité de prélèvement par contact pour cultures selon la revendication 1, caractérisée en ce que la nouvelle bordure est constituée d'une bande démontable (11).

3. Unité de prélèvement par contact pour cultures selon la revendication 2, caractérisée en ce que la bande démontable (11) est constituée d'une bande à arracher pourvue d'une languette (13).

**Claims**

1. Pull-off culture receptacle with a dish which has a flange running along its edge, the space formed within the flange of the dish being intended for filling with agar nutrient medium or being filled therewith, characterised in that, over the upper edge (12), a further peripheral edge (11) projecting upwards is provided, in that the entire space formed by the flange (9) and the further peripheral edge (11) is intended for filling with agar nutrient medium (3), or is filled therewith, and in that the further peripheral edge (11) can be removed.

2. Pull-off culture receptacle according to Claim 1, characterised in that the further peripheral edge is designed as a removable strip (11).

3. Pull-off culture receptacle according to Claim 2, characterised in that the removable strip (11) is designed as a tear-off strip provided with a tab (13).

0 013 748

Fig.1

Fig.2

Fig.3

0 013 748

Fig. 4

Fig. 5

Fig. 6

2